# EUROPÄISCHE PATENTANMELDUNG

(11) **EP 4 066 811 A1**
(43) Veröffentlichungstag der Anmeldung: **05.10.2022**
(21) Anmeldenummer: 21166451.1
(22) Anmeldetag: 31.03.2021
(51) Int. Cl.: A61K 6/30, A61K 6/61, A61C 3/00

(54) **LAGERSTABILES DENTALADHÄSIVSET ZUR ANWENDUNG MIT PEROXIDISCH UND HYDROPEROXIDISCH REDOXINITIIERTEN KOMPOSITEN**

(71) Anmelder: Ivoclar Vivadent AG, 9494 Schaan (LI)
(72) Erfinder: Köhler, Thomas, 78479 Reichenau (DE); Bock, Thorsten, 6806 Tosters (AT); Barbisch, Michael, 6833 Klaus (AT)
(74) Vertreter: Uexküll & Stolberg

(57) **Zusammenfassung**

Dentaladhäsivset, das ein Adhäsiv und einen Applikator enthält, der mit einem Vanadium(IV)salz beschichtet ist.

## Beschreibung

Die vorliegende Erfindung betrifft Dentaladhäsive, die sich zur Anwendung mit peroxidisch und hydroperoxidisch redoxinitiierten Kompositen eignen und die eine hohe Lagerstabilität aufweisen.

In der direkten und indirekten Restaurationstherapie ist die adhäsive Befestigung methacrylatbasierter Kompositmaterialien weit verbreitet. Hierbei wird auf das zu versorgende Zahnhartgewebe zunächst ein methacrylatbasierter Haftvermittler ("Adhäsiv") aufgetragen. Es existieren verschiedene Typen solcher Adhäsive, die sich neben der Anzahl der Komponenten vornehmlich in der Art der Anwendung unterscheiden. Manche Adhäsive erfordern eine Vorbehandlung der Zahnhartsubstanz mit Phosphorsäure (Total Etch oder Etch & Rinse Verfahren), andere Adhäsive weisen selbstätzende Eigenschaften auf (Self Etch Verfahren). Adhäsive bestehen meist aus Mischungen verschiedener Methacrylate ("Monomere") in Lösemitteln. Die Monomere können eine oder mehrere Methacrylatgruppen und weitere funktionelle Gruppen enthalten. Weitere funktionelle Gruppen dienen der Bereitstellung besonderer Produkteigenschaften, wie z.B. Carbonsäure- oder Phosphorsäureestergruppen der Selbstätzung und der Haftvermittlung auf Zahnhartgewebe. Die Adhäsive können zusätzlich Verarbeitungshilfsmittel (z.B. Rheologieadditive), Stabilisatoren (Radikalfänger), sowie Photoinitiatoren und ggf. Teile von Redoxinitiatorsystemen enthalten.

Nach dem Auftragen des Adhäsivs werden das oder die Lösemittel durch Belüften mit einem Luftstrom entfernt und die entstehende Adhäsivschicht üblicherweise durch Belichtung polymerisiert. Auf die Oberfläche des Adhäsivs wird dann in weiteren Schritten der Füllstoff und Methacrylate enthaltende Komposit aufgetragen. Bei Härtung des Komposits copolymerisieren Adhäsiv und Komposit und führen so zum gewünschten mechanisch belastbaren Verbund.

Im Falle der direkten Restaurationstherapie dient der Komposit als Füllungsmaterial ("Füllungskomposit"). Er wird als fließ- oder stopffähige Paste direkt in die Kavität auf die gehärtete Adhäsivschicht aufgebracht und so modelliert, dass die gewünschte Formgebung erzielt wird. Bei der indirekten Restaurationstherapie dient der Komposit zur Befestigung vorgefertigter Restaurationsmaterialien aus Keramik, Metall oder vorpolymerisiertem Komposit, wie z.B. Kronen, Inlays, Onlays oder Veneers ("Befestigungskomposit"). Der Befestigungskomposit überbrückt hier das Volumen zwischen der Adhäsivoberfläche und dem Restaurationsmaterial.

Die Aushärtung von Füllungs- oder Befestigungskomposits erfolgt entweder durch photochemische oder durch eine lichtunabhängige redoxchemische Radikalbildung. In der direkten Restaurationstherapie ist die Lichthärtung sowohl von Adhäsiv und Komposit üblich, da die dort eingesetzten Materialien in der Regel ausreichend transluzent sind. In der indirekten Restaurationstherapie sind jedoch auch gering transluzente Materialien, wie z.B. Oxidkeramiken, oder gänzlich lichtundurchlässige Materialien, wie z.B. Metalle, verbreitet. In solchen Fällen kann in den Komposit nicht die für eine photochemische Polymerisation erforderliche Lichtmenge eingebracht werden, was z.B. zu fehlender oder stark herabgesetzter mechanischer Belastbarkeit führt. Daher werden für die indirekte Restaurationstherapie bevorzugt redoxchemisch polymerisierende Befestigungskomposits verwendet.

Die bei Befestigungskomposits üblicherweise eingesetzte redoxinitiierte Polymerisation beruht auf den Redoxsystemen Peroxid/Amin ("peroxidisch initiierter Komposit") oder Hydroperoxid/Thioharnstoff ("hydroperoxidisch initiierter Komposit"). Hydroperoxidisch initiierte Komposite enthalten häufig zusätzlich eine Übergangsmetallverbindung. Beide Redoxsysteme sind weit verbreitet, werden von den Herstellern aber in der Regel nicht ausgewiesen und können auch vom Fachmann nicht ohne chemische Analyse des Komposits unterschieden werden. Ein Zahnarzt kann also nicht erkennen, ob ein Befestigungskomposit durch ein peroxidisches oder hydroperoxidisches Redoxsystem polymerisiert. Dies ist problematisch, weil Adhäsiv und Komposit aufeinander abgestimmt werden müssen.

Ein weiteres Problem ist, dass die in Befestigungskompositen eingesetzten Redoxinitiatoren, insbesondere das Peroxid/Amin-System, pH-empfindlich sind und durch selbstätzende, z.B. carbon- oder phosphorsaure, Adhäsive inhibiert werden, was eine Abnahme der Verbundwirkung zwischen Adhäsiv und Komposit bewirkt. Um dieser Inhibierung der Initiatoren des Komposits vorzubeugen, wird vom Adhäsivhersteller in der Regel empfohlen, das Adhäsiv vor dem Auftragen des Befestigungskomposits mit Licht zu härten. Auf diese Weise werden die Säuregruppen des Adhäsivs durch die Einbindung in die Polymermatrix in ihrer Mobilität gemindert und die Copolymerisation zwischen Adhäsiv und Komposit gefördert.

Die Vorhärtung der Adhäsive kann bei der indirekten Restaurationstherapie jedoch zu Passungenauigkeiten führen, da die Dicke der Adhäsivschicht oder das Ansammeln von Adhäsiv am Kavitätenboden ("Pooling") für den Anwender schwer kontrollierbar sind. Indirekte Restaurationen werden anhand von Abdrücken oder digitaler Scans des zu restaurierenden Zahns möglichst passgenau vorgefertigt. Die Präzision der Vorfertigung führt jedoch dazu, dass selbst kleine Änderungen der Kavitätengeometrie, die nach der Abdrucknahme oder nach dem Scannen vorgenommen werden, wie z.B. durch das Aufbringen einer Adhäsivschicht, ein Verklemmen der Restauration beim Einsetzen bewirken können. Der Einsatz lichtgehärteter Adhäsive in der indirekten Restaurationstherapie stellt daher eine potentielle Fehlerquelle dar.

Die EP 0 006 757 A2 offenbart zweikomponentige dentale Füllungskomposite, die ein stabförmiges Mischwerkzeug und eine pastenförmige Komponente enthalten. Die Härtung der Komposite wird durch ein zweiteiliges Initiatorsystem aus Peroxid und Amin ausgelöst. Eine Initiatorkomponente ist in der Paste gelöst, die andere ist auf das Mischwerkzeug aufgebracht. Zur Aktivierung wird die Paste mit dem Mischwerkzeug gerührt.

Die EP 0 923 924 A2 offenbart ein zahnmedizinisches Klebstoffset, das ein radikalisch polymerisierbares Monomer mit Säuregruppen, einen Photoinitiator und/oder ein Peroxid, ein wasserlösliches organischen Lösungsmittel, Sulfin- oder Barbitursäure und Wasser enthält. Die Sulfin- oder Barbitursäure kann auf einen Applikator aufgebracht werden.

Die WO 2015/181227 A1 betrifft Mikro-Applikatoren, die zumindest teilweise mit Metall, Metall enthaltenden Verbindungen oder metallorganischen Verbindungen und ggf. zusätzlich mit weiteren Additiven beschichtet sind. Das Metall ist vorzugsweise aus Eisen, Kobalt, Nickel, Ruthenium, Rhodium, Palladium, Osmium, Iridium, Platin, Silber, Kupfer, Zinn und Zink ausgewählt. Als weiter Additive werden u.a. Barbitursäure, Sulfinsäuren, Carbonsäuren und Aminsalze genannt. Bei der Verwendung dieser Applikatoren zum Aufbringen dentaler Adhäsive sollen im Vergleich zu unbeschichteten Applikatoren verbesserte Haftwerte erzielt werden.

Die DE 199 56 705 A1 offenbart pinselförmige Applikatoren für dentale Anwendungen, die mit einem Katalysator, z.B. mit Benzolsulfinsäure, und ggf. einem Amin wie Diethanol-p-toluidin beschichtet sind. Zur Anwendung werden die Applikatoren z.B. in ein dentales Adhäsiv eingetaucht und das Adhäsiv dann mit dem Applikator auf den Zahn aufgebracht. Hierbei lösen sich der Katalysator und das Amin in dem Adhäsiv und bewirken die Härtung. Da Katalysator und Amin in trockener Form vorliegen, sollen die Applikatoren besonders stabil sein. Mit diesem System werden optimale Ergebnisse nur mit peroxidhaltigen Kompositen erzielt, nicht aber mit hydroperoxidhaltigen Kompositen, die sich wegen ihrer deutlich besseren Lagerfähigkeit immer weiter durchsetzen.

Aus der WO 2019/211724 A2 sind einkomponentige Dentaladhäsive bekannt, die eine ionische Übergangsmetallverbindung enthalten. Die Adhäsive sollen sich zur Anwendung mit Kompositen eignen, die Peroxide, Peroxyester, Diacylperoxide oder Persulfate als Oxidationsmittel enthalten. Obwohl diese Zusammensetzungen Stabilisatoren zur Verbesserung der Lagerstabilität enthalten, ist ihre Stabilität für kommerzielle Zwecke unzureichend.

Der Erfindung liegt die Aufgabe zugrunde, ein Adhäsiv zur Verfügung zu stellen, das sich für dentale Zwecke eignet und das die oben beschriebenen Unzulänglichkeiten nicht aufweist. Insbesondere soll das Adhäsiv lagerstabil sein und eine hohe Anwendungsdungsicherheit bieten. Das Adhäsiv soll sich zur Anwendung mit peroxidisch und hydroperoxidisch redoxinitiierten Kompositen sowie für direkte und indirekte Restaurationen eignen.

Erfindungsgemäß wird diese Aufgabe durch ein Dentaladhäsivset gelöst, das ein Adhäsiv und einen Applikator enthält. Das Dentaladhäsivset ist dadurch gekennzeichnet, dass der Applikator mit mindestens einem Vanadium(IV)salz beschichtet ist.

Applikator und Adhäsiv sind vor der Anwendung räumlich voneinander getrennt. Zur Anwendung wird das Adhäsiv mit dem beschichteten Applikator in Kontakt gebracht. Dabei löst sich das Vanadiumsalz aus der Beschichtung und wird mit dem Adhäsiv vermischt. Anschließend wird das Adhäsiv mit dem Applikator auf die Zahnoberfläche aufgetragen und danach ein hydroperoxid- oder peroxidhaltiger Komposit auf die getrocknete Adhäsivschicht aufgebracht. Nach dem Aufbringen des Komposits diffundiert das Hydroperoxid bzw. das Peroxid aus dem Komposit in das Adhäsiv und bildet dort durch Reaktion mit dem Vanadiumsalz freie Radikale, die die radikalische Polymerisation starten.

Erfindungsgemäß bevorzugte Vanadium(IV)salze sind Vanadylacetylacetonat, Vanadyloxalat, Vanadylmaltolat und Vanadylpicolinat, besonders bevorzugt sind Vanadylacetylacetonat, Vanadyloxalat und Vanadylpicolinat, und ganz besonders bevorzugt ist Vanadyloxalat. Diese Salze bewirken eine effektive Zersetzung von Peroxiden und insbesondere von Hydroperoxiden und ergeben so einen besonders festen Verbund zwischen den erfindungsgemäßen Adhäsiven und Kompositen. Die Vanadium(IV)salze zeichnen sich gegenüber Cu(II)-Salzen dadurch aus, dass sie auch unter sauren Bedingungen, d.h. beispielsweise in Anwesenheit von aciden Monomeren, aktiv sind.

Die Vanadium(IV)-Verbindungen werden bei der Reaktion mit Peroxiden und Hydroperoxiden zu Vanadium(V) oxidiert. Der Applikator enthält daher vorzugsweise auch ein Reduktionsmittel. Durch das Reduktionsmittel wird das gebildete Vanadium(V) wieder zu Vanadium(IV) reduziert und steht damit für die weitere Reaktion zur Verfügung. Das Reduktionsmittel verhindert außerdem wirksam eine Inaktivierung der Vanadium(IV)salze durch Reaktion mit Luftsauerstoff, beispielsweise während der Applikatorherstellung. Ein bevorzugtes Reduktionmittel ist Ascorbinsäure. Bevorzugte Kombinationen von Vanadiumsalz und Reduktionsmittel sind Vanadylpicolinat und Ascorbinsäure sowie Vanadyloxalat und Ascorbinsäure. Das erfindungsgemäß als Vanadium(IV)salz besonders bevorzugte Vanadyl(IV)oxalat kann aufgrund der reduzierenden Eigenschaften seines Anions auch ohne ein zusätzliches Reduktionsmittel eingesetzt werden.

Neben der Ascorbinsäure selbst können vorteilhaft auch Derivate der Ascorbinsäure verwendet werden. Bevorzugter Derivate sind Salze, Ester und Ether der Ascorbinsäure. Bevorzugte Salze sind die Alkali- und Erdalkalimetallsalze wie Natriumascorbat und Magnesiumascorbat. Bevorzugte Ester sind C₈-C₁₈-Fettsäureester, insbesondere Palmitoylascorbat. Bevorzugte Ether sind C₁-C₄-Alkylether, besonders bevorzugt 3-O-C₁-C₄-Alkylether, ganz besonders bevorzugt 3-O-Ethylascorbinsäure.

Das Reduktionsmittel kann in derselben Schicht wie das Vanadium(IV)salz oder in einer separaten Schicht vorhanden sein. Erfindungsgemäß ist es bevorzugt, dass sich das Reduktionsmittel und das Vanadium(IV)salz in derselben Schicht befinden.

Der erfindungsgemäße Applikator ist vorzugsweise zusätzlich mit einer Sulfinsäure und/oder einem Sulfinsäurederivat beschichtet. Sulfinsäuren und Sulfinsäurederivate bilden zusammen mit Peroxiden freie Radikale, und die zusätzliche Beschichtung der Applikatoren mit mindestens einer Sulfinsäure oder einem Sulfinsäurederivat verbessert daher insbesondere den Verbund der erfindungsgemäßen Adhäsive mit peroxidisch initiierten Kompositen. Bevorzugte Sulfinsäuren sind Benzolsulfinsäure und 4-Methylbenzolsulfinsäure. Bevorzugte Sulfinsäurederivate sind Sulfinsäureester und besonders Sulfinsäuresalze. Besonders bevorzugte Salze sind die Alkalimetallsalze, ganz besonders bevorzugt sind die Alkalimetallsalze der Benzolsulfinsäure und der 4-Methylbenzolsulfinsäure, insbesondere Natriumbenzolsulfinat oder Lithiumbenzolsulfinat. Vanadyl(IV)oxalat zeichnet sich dadurch aus, dass es auch ohne Sulfinsäure oder Sulfinsäurederivat sowohl mit peroxidhaltigen als auch mit hydroperoxidhaltigen Kompositen hohe Haftwerte erzielt. Gemäß einer Ausführungsform der Erfindung sind daher Applikatoren, die mit Vanadyl(IV)oxalat beschichtet sind und die kein Reduktionsmittel, keine Sulfinsäure und kein Sulfinsäurederivat enthalten, besonders bevorzugt.

Im Folgenden wird der Einfachheit halber meist nur von Ascorbinsäure oder Sulfinsäure gesprochen. In beiden Fällen sind aber, wenn nicht anders angegeben, auch die jeweiligen Ester, Salze und Derivate gemeint.

Die Beschichtung der erfindungsgemäßen Applikatoren kann neben den genannten Stoffen weitere Additive enthalten, insbesondere ein oder mehrere Rheologieadditive. Rheologieadditive werden in Thixotropiermittel und Verdicker unterschieden. Als Thixotropiermittel eignen sich besonders pyrogene und Fällungskieselsäuren. Besonders bevorzugt sind Kieselsäuren mit einem Primärpartikeldurchmesser von 5 bis 500 nm. Besonders bevorzugt sind Kieselsäuren mit sphärischen Partikeln. Als Verdicker sind Polymere bevorzugt, insbesondere Polyacrylsäure, Polyitaconsäure, Polyvinylakohol, Polystyrol und deren Derivate sowie Copolymere derselben. Unter Derivaten der betreffenden Polymere werden hier durch Anbindung von Ester- oder Ethergruppen modifizierte Abkömmlinge der jeweiligen Polymere verstanden. Von besonderem Interesse im erfindungsgemäßen Sinne sind methacrylatmodifizierte Polyacryl- und Polyitaconsäure. Die hier eingesetzten Polymeren haben vorzugsweise eine gewichtsmittlere Molmasse von 5000 bis 100.000 g/mol. Die Molmasse der Polymere wird vorzugsweise mittels Dampfdruckosmometrie, Ebullioskopie oder Kryoskopie bestimmt.

Die Beschichtung der erfindungsgemäßen Applikatoren enthält vorzugsweise keine Amine, insbesondere keine N,N-Dialkyl-Arylamine, wie z.B. Chivacure EPD, N,N-Dimethyl-Ethyl-p-aminobenzoat oder DABA, N,N-Diethylamin-3,5-di-tert-butylaniline, oder aliphatische Amine, wie z.B. Dimethylaminoethylmethacrylat, DMAEMA, denn es wurde gefunden, dass Amine die Schmelzhaftung der Adhäsive beeinträchtigen können.

Als Applikatoren eignen sich besonders die zur Adhäsivapplikation verbreiteten Mikropinsel ("Microbrush"), d.h. kleine Pinsel, bei denen ein Ende eines stabförmigen Pinselschafts mit einer für das Auftragen von Flüssigkeiten geeigneten Beschichtung (Beflockung, Schwamm, Borsten, Gummilamellen etc.) versehen ist. Geeignete Applikatoren werden beispielsweise in der DE 199 56 705 A1 beschrieben. Ebenfalls anwendbar sind Spritzen- oder Aufsteckkanülen, bei denen ein geeignetes Metall- oder Kunststoffröhrchen an einem Ende mit einer für das Auftragen von Flüssigkeiten geeigneten Beschichtung (Beflockung, Schwamm, Borsten, Gummilamellen etc.) versehen ist, und das am anderen Ende eine mit einem Flüssigkeitsbehälter, z.B. einer Injektionsspritze, kompatible Aufnahme (z.B. Luerlock) aufweist. Der Applikator kann mit einem Flüssigkeitsbehälter verbunden werden, der zur Aufnahme des Adhäsivs dient, wie z.B. einem Stiftapplikator.

Es wurde überraschend gefunden, dass die Beschichtungsreihenfolge beim Aufbringen der Schichten auf den Applikator einen entscheidenden Einfluss auf die spätere Haftung zwischen Adhäsiv und Komposit hat. Erfindungsgemäß wird der Applikator vorzugsweise erst mit einem Vanadium(IV)salz oder einer Mischung von Vanadium(IV)salzen beschichtet, wobei diese erste Schicht vorzugsweise auch ein Reduktionsmittel enthält. Erst danach wird der Applikator mit einer Sulfinsäure oder einer Mischung von Sulfinsäuren beschichtete, so dass eine zweite (äußere) Schicht gebildet wird. Auf die beschriebene Weise beschichtete Applikatoren ergeben mit peroxidisch initiierten Kompositen deutlich bessere Haftwerte als Applikatoren, bei denen Sulfinsäure(n) und Vanadium(IV)salz(e) in umgekehrter Reihenfolge oder gleichzeitig auf den Applikator aufgebracht wurden.

Gegenstand der Erfindung sind auch ein Verfahren zur Herstellung beschichteter Applikatoren sowie die erhaltenen Applikatoren. Erfindungsgemäß erfolgt die Beschichtung der Applikatoren vorzugsweise in einem Tauchprozess, der die folgenden Schritte umfasst:
(1) Bereitstellen einer Lösung eines oder mehrerer Vanadium(IV)salze in einem geeigneten Lösungsmittel,
(2) ein- oder mehrfaches Eintauchen eines Applikators in die Lösung aus Schritt (1), vorzugsweise für jeweils mindestens 5 Sekunden, bevorzugt 10 bis 60 Sekunden,
(3) Trocknen des Applikators aus Schritt (2), vorzugsweise bei einer Temperatur von ≤ 70°C und bevorzugt für maximal 15 Minuten, und vorzugsweise
(4) Bereitstellen einer Lösung einer oder mehrerer Sulfinsäuren und/oder Sulfinsäurederivate in einem geeigneten Lösungsmittel,
(5) ein- oder mehrfaches Eintauchen des Applikators in die Lösung aus Schritt (4), vorzugsweise für jeweils mindestens 5 Sekunden, bevorzugt 10 bis 60 Sekunden, und
(6) Trocknen des Applikators aus Schritt (5), vorzugsweise bei einer Temperatur von ≤ 70°C für bevorzugt für maximal 15 Minuten.

Die in Schritt (1) verwendete Lösung enthält neben dem oder den Vanadium(IV)salzen vorzugsweise auch ein Reduktionsmittel, besonders bevorzugt Ascorbinsäure. Zur Erhöhung der Beladung des Applikators können die Tauchschritte jeweils ein- oder mehrfach durchgeführt werden. Sollte der Applikator in Schritt (2) und/oder in Schritt (5) mehrfach in die Lösung aus Schritt (1) bzw. Schritt (4) eingetaucht werden, wird er vorzugsweise nach jedem Eintauchen getrocknet, vorzugsweise unter den oben genannten Bedingungen. Eine durch mehrfaches Eintauchen in dieselbe Lösung und ggf. anschließendes Trocknen gebildete Schicht wird erfindungsgemäß als eine (1) Schicht angesehen. Die Schicht wird in diesem Fall durch mehrere Lagen gebildet.

Sollte der Applikator mit mehr als einer Sulfinsäure und/oder mehr als einem Sulfinsäurederivat beschichtet werden, kann Schritt (4) in mehrere separate Schritte aufgespalten werden, indem der Applikator zunächst in eine Lösung mit einer ersten Sulfinsäure oder einem ersten Sulfinsäurederivat und danach in eine weitere Lösung mit einer zweiten Sulfinsäure oder einem zweiten Sulfinsäurederivat eingetaucht wird. Bei mehr als zwei Sulfinsäuren oder Sulfinsäurederivaten kann die Anzahl der Lösungen entsprechend angepasst werden. Auch hier wird der Applikator vor dem Eintauchen in eine weitere Lösung vorzugsweise getrocknet. Gleiches gilt sinngemäß dann, wenn der Applikator mit mehr als einem Vanadiumsalz beschichtet wird. Wenn der Applikator nur mit einem oder mehreren Vanadium(IV)salzen beschichtet werden soll, können die Schritte (4) bis (6) entfallen.

Zur Vermeidung wiederholter Tauchschritte kann die Viskosität der Beschichtungslösungen durch Zugabe von Rheologieadditiven erhöht werden, so dass beim Eintauchen eine größere Menge der Lösung an dem Applikator haften bleibt.

Vorzugsweise wird der Applikator in den Schritten (2) und (5) nur teilweise in die Lösung aus Schritt (1) eingetaucht, so dass nur ein Teil des Applikators beschichtet wird und der Rest unbeschichtet bleibt. Der unbeschichtete Teil des Applikators kann z.B. als Griff zum Anfassen des Applikators dienen. Es ist jedoch auch möglich, den Applikator ganz zu beschichten und später mit einem geeigneten Griff zu verbinden.

Die Konzentrationen der Lösungen und ggf. die Zahl der Eintauchschritte werden so gewählt, dass auf dem Applikator solche Mengen der Komponenten abgeschieden werden, die zur Härtung der aufzubringenden Menge des Adhäsivs geeignet sind.

Ein bevorzugtes Lösungsmittel zur Herstellung der Vanadium(IV)salzlösung in Schritt (1) ist Methanol, weil hierin eine zur Beschichtung vorteilhafte Konzentration an Vanadiumsalz erzielt werden kann. Außerdem ist das Lösungsmittel nach dem Beschichten leicht entfernbar. Auch die zur Stabilisierung des Vanadium(IV)salzes ggf. verwendeten Reduktionsmittel, wie z.B. Ascorbinsäure, sind in Methanol gut löslich. Vorzugsweise beträgt die Vanadium(IV)-salzkonzentration 0,5 bis 5 Gew.-%, besonders bevorzugt 1 bis 2 Gew.-%. Die Konzentration des Reduktionsmittels beträgt vorzugsweise 0,5 bis 5 Gew.-%, besonders bevorzugt ca. 1 Gew.-%. Ganz besonders bevorzugt sind Lösungen, die ca. 2 Gew.-% Vanadylpicolinat und ca. 1 Gew.-% Ascorbinsäure oder 1 Gew.-% Vanadyloxalat enthalten.

Alle Gew.-%- und ppm-Angaben hierin beziehen sich, wenn nicht anders angegeben, jeweils auf die Gesamtmasse der jeweiligen Zusammensetzung.

Zur Herstellung der Sulfinsäurelösung in Schritt (4) ist Ethanol als Lösungsmittel bevorzugt. Ethanol hat den Vorteil, dass Sulfinsäuren und Sulfinsäuresalze wie Natriumbenzolsulfinat gut, Vanadiumsalze und Reduktionsmittel aber nur relativ schlecht darin löslich sind, so dass die zuvor aufgebrachte Schicht von Vanadiumsalz und Reduktionsmittel nicht oder nicht in einem funktionsrelevanten Umfang wieder abgelöst wird. Vorzugsweise beträgt die Sulfinsäure- oder Sulfinsäuresalzkonzentration 1 bis 10 Gew.-%, besonders bevorzugt ca. 2 Gew.-%. Ganz besonders bevorzugt ist eine Lösung, die ca. 2 Gew.-% Natriumbenzolsulfinat enthält.

Generell werden die Lösungsmittel zur Herstellung der Beschichtungslösungen so gewählt, dass die in einem Verfahrensschritt aufgebrachte Schicht im nachfolgenden Schritt nicht wieder abgelöst wird. Da die Löslichkeit unterschiedlicher Sulfinsäuren oder Vanadiumsalze variieren kann, kann es erforderlich sein, die Wahl der Lösungsmittel entsprechend anzupassen.

Das Trocknen in den Schritten (3) und (6) erfolgt vorzugsweise jeweils in einem Temperaturbereich von Raumtemperatur (20°C) bis ≤ 70°C, besonders bevorzugt bei 40°C bis 70°C, ganz besonders bevorzugt bei ca. 40°C. Die Trocknungszeit beträgt vorzugsweise 5 bis 10 Minuten. Das Trocknen kann z.B. in einem Trockenschrank erfolgen. Das Trocknen bewirkt, dass die Komponenten an dem Applikator haften bleiben.

Durch die obige Schrittreihenfolge werden eine hohe Lagerstabilität und eine optimale Haftung am Zahn erzielt, sowohl bei der Verwendung des Adhäsivs mit peroxidisch als auch bei der Verwendung mit hydroperoxidisch initiierten Kompositen.

Ein besonderer Vorteil der erfindungsgemäßen Applikatoren ist, dass die Schichten auf die beschriebene Weise direkt nacheinander aufgebracht werden können, ohne dass die Verwendung einer Trennschicht zwischen Vanadiumsalz- und Sulfinsäure- bzw. Sulfinsäurederivatschicht erforderlich ist. Die Applikatoren weisem gemäß einer bevorzugten Ausführungsform daher nur eine Vanadiumsalz- und ggf. eine Sulfinsäure- bzw. Sulfinsäurederivatschicht auf.

Vanadyl(IV)salze werden vorzugsweise in einer Menge von 30 bis 100 µg, besonders bevorzugt 40 bis 90 µg und ganz besonders bevorzugt 50 bis 80 µg auf den Applikator aufgebracht. Alle Angaben beziehen sich auf die Menge pro Applikator. Erfindungsgemäß sind Microbrushes, die im Mittel mit 50 bis 80 µg Vanadyl(IV)oxalat beschichtet sind, und Aufsteckkanülen, die im Mittel mit ca. 50 µg Vanadyl(IV)oxalat beschichtet sind, besonders bevorzugt.

Sulfinsäure und Sulfinsäurederivate werden vorzugsweise in einer Menge von 60 bis 200 µg, besonders bevorzugt 80 bis 180 µg und ganz besonders bevorzugt 100 bis 160 µg pro Applikator aufgebracht.

Reduktionsmittel wie Ascorbinsäure und Ascorbinsäurederivte werden vorzugsweise in einer Menge von 15 bis 50 µg, besonders bevorzugt 20 bis 45 µg und ganz besonders bevorzugt 25 bis 40 µg pro Applikator aufgebracht.

Das erfindungsgemäße Adhäsiv enthält vorzugsweise
(a) mindestens ein radikalisch polymerisierbares acides Monomer,
(b) mindestens ein radikalisch polymerisierbares Monomer ohne acide Gruppen,
(c) mindestens ein mit Wasser mischbares organisches Lösungsmittel und
(d) Wasser.

Außerdem enthält das Adhäsiv vorzugsweise auch
(e) ein oder mehrere Thixotropiermittel und/oder Füllstoffe und/oder
(f) einen oder mehrere Stabilisatoren.

Weiterhin kann das Adhäsiv als Komponente (g) einen oder mehrere Initiatoren für die photochemisch ausgelöste radikalische Polymerisation (Photoinitiator) und/oder einen Verdicker als Komponente (h) enthalten. Das Adhäsiv enthält vorzugsweise keine Übergangsmetallverbindungen und insbesondere keine Vanadium(IV)salze.

Das erfindungsgemäße Adhäsiv enthält als Bestandteil (a) vorzugsweise ein oder mehrere säuregruppenhaltige radikalisch polymerisierbare Monomere (Haftmonomere; acide Monomere). Bevorzugt sind acide Monomere, die als Säuregruppen mindestens eine Carbonsäuregruppe, Sulfonsäuregruppe, Phosphonsäuregruppe, Phosphorsäureestergruppe, vorzugsweise eine Monohydrogenphosphatgruppe oder Dihydrogenphosphatgruppe, aufweisen. Ganz besonders bevorzugt sind acide Monomere mit Dihydrogenphosphorsäureestergruppen.

Bevorzugte Monomere mit Carbonsäuregruppen sind Maleinsäure, Acrylsäure, Methacrylsäure, 2-(Hydroxymethyl)acrylsäure, 4-(Meth)acryloyloxyethyltrimellitsäure, 10-Methacryloyloxydecylmalonsäure, N-(2-Hydroxy-3-methacryloyloxypropyl)-N-phenylglycin bzw. 4-Vinylbenzoesäure.

Bevorzugte Monomere mit Phosphonsäuregruppen sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentylphosphonsäure, 2-[4-(Dihydroxyphosphoryl)-2-oxa-butyl]-acrylsäure oder 2-[4-(Dihydroxyphosphoryl)-2-oxabutyl]-acrylsäureethyl- und -2,4,6-trimethylphenylester sowie β-Ketophosphonsäuremethacrylate, wie z.B. 9-Methacryloyloxy-2-oxononylphosphonsäure. Besonders bevorzugt sind die in WO 2014/202176 A1 beschriebenen β-Ketophosphonsäuremethacrylate.

Bevorzugte Monomere mit Phosphorsäureestergruppen sind 2-Methacryloyloxypropylmono-oder -dihydrogenphosphat, 2-Methacryloyloxyethylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)hexyldihydrogenphosphat und 1,3-Bis-(N-acryloyl-N-propylamino)-propan-2-yl-dihydrogenphosphat und 9-Methacryloyloxy-2-oxononylphosphonsäure.

Bevorzugte Monomere mit Sulfonsäuregruppen sind Vinylsulfonsäure, 4-Vinylphenylsulfonsäure oder 3-(Methacrylamido)propylsulfonsäure.

Besonders bevorzugte Säuremonomere sind 4-(Meth)acryloyloxyethyltrimellitsäureanhydrid, 10-Methacryloyloxydecylmalonsäure, 2-Methacryloyloxyethylphosphonsäure, 2-Methacrylamidoethylphosphonsäure, 2-[4-(Dihydroxyphosphoryl)-2-oxa-butyl]-acrylsäure, 2-[4-(Dihydroxyphosphoryl)-2-oxa-butyl]-acrylsäureethyl- oder -2,4,6-trimethylphenylester. 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, 6-(Methacrylamido)hexyldihydrogenphosphat, 1,3-Bis-(N-acryloyl-N-propyl-amino)-propan-2-yl-dihydrogenphosphat und insbesondere 10-Methacryloyloxydecyl-dihydrogenphosphat.

Als Bestandteil (b) enthält das Adhäsiv vorzugsweise ein nicht acides radikalisch polymerisierbares Monomer oder Mischungen radikalisch polymerisierbarer Monomere. Bevorzugt sind (Meth)acrylate, besonders bevorzugt Mischungen aus mono- und polyfunktionellen (Meth)acrylaten, ganz besonders bevorzugt aus mono- und difunktionellen (Meth)acrylaten. Unter Mono(meth)acrylaten werden Verbindungen mit einer, unter di- und polyfunktionellen (Meth)acrylaten Verbindungen mit zwei oder mehr, vorzugsweise 2 bis 4 radikalisch polymerisierbaren Gruppen verstanden. In allen Fällen sind Methacrylate gegenüber den Acrylaten bevorzugt.

Bevorzugte mono- oder polyfunktionelle (Meth)acrylate sind Methyl-, Ethyl-, 2-Hydroxyethyl-, 2-Hydroxypropyl-, n-Butyl-, Benzyl-, Tetrahydrofurfuryl- oder lsobornyl(meth)acrylat, 2-Acetoacetoxyethylmethacrylat, p-Cumyl-phenoxyethylenglycolmethacrylat (CMP-1E), Bis-GMA (ein Additionsprodukt aus Methacrylsäure und Bisphenol-A-diglycidylether), UDMA (ein Additionsprodukt aus 2-Hydroxyethylmethacrylat (HEMA) und 2,2,4-Trimethylhexamethylendiisocyanat), tricyclisches UDMA (V-818; CAS Nr. 1998085-44-1) oder dessen Isomerenmischung (CAS Nr. 106981-29-7),

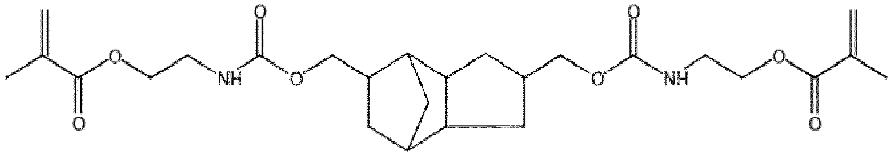

### 2-Propensäure-, 2-methyl-, 1,1'-[(octahydro-4,7-methano-1H-inden-2,5-diyl)bis-(methylenoxycarbonylimino-2,1-ethandiyl)]ester (CAS Nr. 1998085-44-1)

TMX-UDMA (ein Additionsprodukt aus einer Mischung von HEMA und Hydroxypropylmethacrylat (HPMA) mit α,α,α',α'-Tetramethyl-m-xylylendiisocyanat (TMXDI)), Bis(methacryloyloxymethyl)tricyclo[5.2.1.]decan (TCDMA), ethoxy- oder propoxyliertes Bisphenol-A-di(meth)acrylat, wie z.B. das Bisphenol-A-dimethacrylat 2-[4-(3-Methacryloyloxyethoxyethyl)phenyl]-2-[4-(3-methacryloyloxyethyl)phenyl]-propan) (SR-348C) mit 3 Ethoxygruppen oder 2,2-Bis[4-(2-(meth)acryloxypropoxy)phenyl]propan, Di-, Tri- oder Tetraethylenglycoldi(meth)acrylat, Trimethylolpropantri(meth)acrylat, Pentaerythrittetra(meth)acrylat, sowie Glycerindi(meth)acrylatacetat, Glycerindi(meth)acrylat, 1,4-Butandioldi(meth)acrylat, 1,10-Decandioldi(meth)acrylat oder 1,12-Dodecandioldi(meth)acrylat, Glycerintrimethacrylat (GTMA) und Mischungen davon.

Weiter bevorzugt sind N-mono- oder N-disubstituierte Acrylamide, wie z.B. N-Ethylacrylamid, N,N-Dimethylacrylamid, N-(2-Hydroxyethyl)acrylamid oder N-Methyl-N-(2-hydroxyethyl)acrylamid, bzw. N-monosubstituierte Methacrylamide, wie z.B. N-Ethylmethacrylamid oder N-(2-Hydroxyethyl)ethacrylamid sowie N-Vinylpyrrolidon oder Allylether. Diese Monomere zeichnen sich durch eine geringe Viskosität und eine hohe Hydrolysestabilität aus und eignen sich besonders als Verdünnermonomere.

Ebenfalls bevorzugt sind vernetzende Pyrrolidone, wie z.B. 1,6-Bis(3-vinyl-2-pyrrolidonyl)-hexan oder kommerziell zugängliche Bisacrylamide, wie Methylen- oder Ethylenbisacrylamid, bzw. Bis(meth)acrylamide, wie z.B. N,N'-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsäurechlorid synthetisiert werden können. Diese Monomere zeichnen sich durch eine hohe Hydrolysestabilität aus und eignen sich besonders als Vernetzermonomere.

Besonders bevorzugte Monomere sind HEMA, CMP-1E, Bis-GMA, tricyclisches UDMA (V-818; CAS Nr. 1998085-44-1 oder 106981-29-7), UDMA, TMX-UDMA, TCDMA, ethoxy- oder propoxyliertes Bisphenol-A-dimethacrylat, SR-348c, Triethylenglycoldimethacrylat, Glycerindimethacrylat, 1,10-Decandioldimethacrylat (D3MA), Glycerintrimethacrylat (GTMA), N,N'-Diethyl-1,3-bis(acrylamido)-propan, Maleinsäureanhydrid und Mischungen davon. Besonders bevorzugte Mischungen von mono- und difunktionellen (Meth)acrylaten sind Mischungen von HEMA mit Bis-GMA und 1,10-Decandioldimethacrylat oder von HEMA, V-818 und 1,10-Decandioldimethacrylat. Ganz besonders bevorzugt sind Adhäsive, die 18 bis 25 Gew.-% HEMA, 18 bis 25 Gew.-% V-818 und/oder Bis-GMA und 6 bis 10 Gew.-% 1,10-Decandioldimethacrylat enthalten, jeweils bezogen auf die Gesamtmasse des Adhäsivs.

Das erfindungsgemäße Adhäsiv enthält als Bestandteil (c) ein mit Wasser mischbares organisches Lösungsmittel, vorzugsweise Ethanol, Methanol, n-Propanol, Isopropylalkohol, Aceton und Methyl-Ethyl-Keton oder eine Mischung davon.

Bestandteil (d) ist Wasser. Vorzugsweise destilliertes oder entionisiertes Wasser. Das Wasser ist frei von Verunreinigungen und vorzugsweise steril.

Weiterhin kann das Adhäsiv als Bestandteil (e) vorzugsweise auch ein oder mehrere Thixotropiermittel und/oder Füllstoffe enthalten. Bevorzugt sind Adhäsive, die mindestens einen organischen oder besonders bevorzugt anorganischen partikulären Füllstoff oder eine Mischung davon enthalten. Der Füllstoff-Zusatz wird bevorzugt zur Verbesserung der mechanischen Eigenschaften zur Anpassung der Viskosität und zur Optimierung der rheologischen Eigenschaften zugegeben. Bevorzugt sind amorphe kugelförmige Materialien als Füllstoffe auf der Basis von Oxiden, insbesondere SiO₂, wie z.B. pyrogene Kieselsäure oder Fällungskieselsäure (gewichtsmittlere Partikelgröße von 10-1000 nm) sowie Minifüllstoffe, wie Quarz-, Glaskeramik- oder röntgenopake Glaspulver von z.B. Barium- oder Strontiumaluminiumsilikatgläsern (gewichtsmittlere Partikelgröße von 0,01-10 µm, besonders bevorzugt 0,01-1 µm, ganz besonders bevorzugt 0,2-1 µm). Weitere bevorzugte Füllstoffe sind röntgenopake Füllstoffe, wie Ytterbiumtrifluorid oder nanopartikuläres Tantal(V)-oxid bzw. Bariumsulfat oder Mischoxide von SiO₂ mit Ytterbium(III)-oxid oder Tantal(V)-oxid (gewichtsmittlere Partikelgröße von 10-1000 nm). Die erfindungsgemäßen Dentalwerkstoffe enthalten vorzugsweise keine ionenfreisetzenden Füllstoffe, insbesondere keine Ca²⁺- oder Al³⁺-freisetzenden Gläser.

Wenn nicht anders angegeben, handelt es sich bei allen Partikelgrößen um gewichtsmittlere Partikelgrößen (D50-Werte), wobei die Partikelgrößenbestimmung im Bereich von 0,1 µm bis 1000 µm vorzugsweise mittels statischer Lichtstreuung erfolgt, beispielsweise mit einem statischen Laserstreuungs-Partikelgrößen-Analysator LA-960 (Horiba, Japan). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine 0,1 bis 0,5%ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320. Die Messung der Partikelgröße im Bereich von 5 nm bis 0,1 µm erfolgt vorzugsweise durch Dynamische Lichtstreuung (DLS) von wässrigen Partikeldispersionen, vorzugsweise mit einem He-Ne-Laser mit einer Wellenlänge von 633 nm, bei einem Streuwinkel von 90° und bei 25°C, z.B. mit einem Malvern Zetasizer Nano ZS (Malvern Instruments, Malvern UK).

Partikelgrößen kleiner als 0,1 µm können auch anhand von REM- oder TEM-Aufnahmen bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf einem 50 Å dickem Kupfergitter (Maschenweite 300 Mesh) aufgebracht, das mit Kohlenstoff beschichtet ist, und im Anschluss das Lösungsmittel verdampft. Die Partikel werden ausgezählt und der arithmetische Mittelwert berechnet.

Zur Verbesserung des Verbundes zwischen den Füllstoffpartikeln und der vernetzten Polymerisationsmatrix können SiO₂-basierende Füllstoffe mit methacrylatfunktionalisierten Silanen, wie z.B. 3-Methacryloyloxypropyltrimethoxysilan, oberflächenmodifiziert werden.

Als Komponente (f) enthält das Adhäsiv vorzugsweise einen oder mehrere Stabilisatoren. Hierbei handelt es sich um radikalfangende Stoffe zur Verhinderung einer vorzeitigen Polyreaktion. Die Stabilisatoren werden auch als Polymerisationsinhibitoren bezeichnet. Die Inhibitoren bzw. Stabilisatoren verbessern die Lagerstabilität der Materialien.

Bevorzugte Inhibitoren sind Phenole, wie Hydrochinonmonomethylether (MEHQ) oder 2,6-Di-tert.-butyl-4-methyl-phenol (BHT). Phenole werden vorzugsweise in einer Konzentration von 0,001 bis 0,50 Gew.-% verwendet. Weitere bevorzugte Inhibitoren sind Phenothiazin, das 2,2-Diphenyl-1-picrylhydrazyl (DPPH)-, das Galvinoxyl-, das Triphenylmethyl- und das 2,2,6,6-Tetramethyl-piperindin-1-oxyl-Radikal (TEMPO). Diese Inhibitoren werden vorzugsweise in einer Menge von 0,001 bis 0,02 Gew.-% eingesetzt. Eine Polymerisation findet erst dann statt, wenn diese Additive verbraucht sind. Die Mengenangaben beziehen sich jeweils auf die Gesamtmasse des Materials. Vorzugsweise wird eine Mischung von Inhibitoren eingesetzt, die mindestens ein Phenol und mindestens einen der weiteren Initiatoren enthält.

Die Härtung der erfindungsgemäßen Adhäsive erfolgt durch ein Zusammenwirken von Adhäsiv, Applikator und Komposit. Zur Anwendung wird das Adhäsiv mit dem beschichteten Applikator in Kontakt gebracht. Dabei lösen sich das Vanadiumsalz und ggf. die Sulfinsäure aus der Beschichtung und werden mit dem Adhäsiv vermischt. Anschließend wird dieses auf die Schmelz- oder Dentinoberfläche aufgebracht und durch das Entfernen des Lösungsmittels getrocknet. Danach wird ein hydroperoxid- oder peroxidhaltiger Komposit auf die getrocknete Adhäsivschicht aufgebracht. Nach dem Aufbringen des Komposits diffundieren Hydroperoxid bzw. Peroxid aus dem Komposit in das Adhäsiv und lösen dort die Polymerisation aus.

Um bei Bedarf eine zusätzliche Lichthärtung zu ermöglichen, kann das Adhäsiv als Bestandteil (g) einen Photoinitiator für die radikalische Polymerisation enthalten. Bevorzugte Photoinitiatoren sind Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil. Besonders bevorzugte Initiatoren sind Campherchinon und 2,2-Dimethoxy-2-phenyl-acetophenon und ganz besonders bevorzugt α-Diketone in Kombination mit Aminen als Reduktionsmittel, wie z.B. 4-(Dimethylamino)benzoesäureethylester, N,N-Dimethylaminoethylmethacrylat, N,N-Dimethyl-sym.-xylidin oder Triethanolamin. Besonders bevorzugt sind auch Norrish-Typ-I-Photoinitiatoren, vor allem Acyl- der Bisacylphosphinoxide, Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethylgermanium. Dabei lassen sich vorteilhaft auch Mischungen der verschiedenen Photoinitiatoren einsetzen, wie z.B. Dibenzoyldiethylgermanium in Kombination mit Campherchinon und 4-Dimethylaminobenzoesäureethylester. Photoinitiatoren werden insbesondere für den Einsatz des Adhäsivs bei der direkten Füllungstherapie zugegeben.

Weiter bevorzugt sind Adhäsive, die als Rheologieadditv mindestens einen Verdicker (h) enthalten, wobei die oben genannten Verdicker bevorzugt sind, d.h. insbesondere Polyacrylsäure, Polyitaconsäure, Polyvinylakohol, Polystyrol und/oder Derivate oder Copolymere davon. Besonders bevorzugt sind methacrylatmodifizierte Polyacryl- und Polyitaconsäure, z.B. mit Glycidylmethacrylat oder Methacrylsäure-2-isocyanatoethylester modifizierte Polyacrylsäure. Ganz besonders bevorzugt sind Polymere mit einer gewichtsmittleren Molmasse von 5000 bis 100.000 g/mol, insbesondere 5000 bis 50.000 g/mol. Es wurde gefunden, dass die erfindungsgemäß bevorzugten Verdicker neben ihrer eigentlichen Funktion die Haftung der Adhäsive an Zahnhartgewebe verbessern. Verdicker werden vorzugsweise in einer Menge von 0,1 bis 8 Gew.-%, besonders bevorzugt 0,2 bis 7 Gew.-% und ganz besonders bevorzugt 3 bis 6 Gew.-% eingesetzt.

Erfindungsgemäß bevorzugt sind Adhäsive, die zusätzlich mindestens ein Alkalimetallhydroxid enthalten, vorzugsweise NaOH, KOH und/oder RbOH, besonders bevorzugt KOH und/oder RbOH und ganz besonders bevorzugt KOH. Das oder die Alkalimetallhydroxide werden vorzugsweise in einer Gesamtmenge von 3 bis 15 mmol, vorzugsweise 6 bis 12 mmol pro 100 g Adhäsiv eingesetzt.

Gegebenenfalls können die erfindungsgemäßen Zusammensetzungen außerdem weitere Additive enthalten, beispielsweise Aromastoffe, Farbmittel, mikrobizide Wirkstoffe, fluoridionenabgebende Additive, optische Aufheller, Fluoreszenzmittel, Weichmacher, Kettentransferreagenzien und/oder UV-Absorber.

Erfindungsgemäß weist das Adhäsiv vorzugsweise die folgende Zusammensetzung auf:
(a) 1 bis 40 Gew.-%, bevorzugt 2 bis 30 Gew.-% und besonders bevorzugt 5 bis 20 Gew.-% acide radikalisch polymerisierbare(s) Monomer(e),
(b) 1 bis 80 Gew.-%, bevorzugt 1 bis 60 Gew.-% und besonders bevorzugt 5 bis 55 Gew.-% nicht acide radikalisch polymerisierbare(s) Monomer(e),
(c) 0 bis 70 Gew.-%, bevorzugt 5 bis 60 Gew.-% und besonders bevorzugt 10 bis 50 Gew.-% Lösungsmittel,
(d) 0 bis 70 Gew.-%, bevorzugt 5 bis 60 Gew.-% und besonders bevorzugt 8 bis 50 Gew.-% Wasser, und ggf.
(e) 0 bis 20 Gew.-%, bevorzugt 1 bis 15 Gew.-% Füllstoff(e), und ggf.
(f) 0,001 bis 0,35 Gew.-%, bevorzugt 0,03 bis 0,30% Gew.-5 und besonders bevorzugt 0,05 bis 0,25 Gew.-% Stabilisator(en).

Die zur Einstellung der Viskosität und der rheologischen Eigenschaften ggf. verwendeten Thixotropiermittel sind in der Füllstoffmenge berücksichtigt.

Gemäß einer optionalen Ausführungsform kann das Adhäsiv außerdem
(g) 0,01 bis 10 Gew.-%, bevorzugt 0,1 bis 5,0 Gew.-% und besonders bevorzugt 0,5 bis 4,5 Gew.-% Photoinitiator(en) und/oder
(h) 0,1 bis 8 Gew.-%, besonders bevorzugt 0,2 bis 7 Gew.-% und ganz besonders bevorzugt 3 bis 6 Gew.-% Verdicker und/oder
   6 bis 12 mmol KOH pro 100 g Adhäsiv enthalten.

Besonders bevorzugt hat das Adhäsiv die folgende Zusammensetzung:
(a) 1 bis 30 Gew.-%, bevorzugt 2 bis 30 Gew.-% und besonders bevorzugt 5 bis 20 Gew.-% acide radikalisch polymerisierbare(s) Monomer(e),
(b) 1 bis 80 Gew.-%, bevorzugt 1 bis 60 Gew.-% und besonders bevorzugt 5 bis 55 Gew.-% nicht acide radikalisch polymerisierbare(s) Monomer(e),
(c) 0 bis 70 Gew.-%, bevorzugt 5 bis 60 Gew.-% und besonders bevorzugt 10 bis 50 Gew.-% Lösungsmittel, vorzugsweise Methanol oder Ethanol,
(d) 0 bis 70 Gew.-%, bevorzugt 5 bis 60 Gew.-% und besonders bevorzugt 8 bis 50 Gew.-% Wasser und ggf.
(e) 0 bis 20 Gew.-% Füllstoff.

Außerdem kann das Adhäsiv ggf.
(f) 0,001 bis 0,35 Gew.-%, bevorzugt 0,03 bis 0,30 Gew.-% und besonders bevorzugt 0,05 - 0,25 Gew.-% Stabilisator(en) und/oder
(g) 0,01 bis 10 Gew.-%, bevorzugt 0,1 bis 4,5 Gew.-% und besonders bevorzugt 0,5 bis 4,0 Gew.-% an Photoinitiator(en) und/oder
(h) 0,1 bis 8 Gew.-%, besonders bevorzugt 0,2 bis 7 Gew.-% und ganz besonders bevorzugt 3 bis 6 Gew.-% Verdicker enthalten.

Ganz besonders bevorzugt hat das Adhäsiv die folgende Zusammensetzung:
(a) 10 bis 20 Gew.-% acide radikalisch polymerisierbare(s) Monomer(e), vorzugsweise 10-Methacryloyloxydecyl-dihydrogenphosphat,
(b) 30 bis 55 Gew.-% nicht acide radikalisch polymerisierbare(s) Monomer(e), vorzugsweise 18 bis 25 Gew.-% monofunktionelles Monomer, insbesondere HEMA, und 24 bis 35 Gew.-% difunktionelles Monomer, insbesondere Bis-GMA, 1,10-Decandioldimethacrylat, V-818 oder eine Mischung davon,
(c) 10 bis 15 Gew.-% Lösungsmittel, vorzugsweise Ethanol,
(d) 8 bis 15 Gew.-% Wasser,
(e) 3 bis 6 Gew.-% Füllstoff, vorzugsweise pyrogene Kieselsäure,
(f) 0,1 bis 0,25 Gew.-% Stabilisator(en),
(g) 1,0 bis 4,0 Gew.-% an Photoinitiator(en),
(h) 3 bis 6 Gew.-% Verdicker, vorzugsweise methacrylatmodifizierte Polyacrylsäure mit einer gewichtsmittleren Molmasse von 5000 bis 50.000 g/mol, und
   6 bis 12 mmol KOH pro 100 g Adhäsiv.

Wenn nicht anders angegeben beziehen sich alle Mengenangaben auf die Gesamtmasse des Adhäsivs. Die einzelnen Mengenbereiche können separat gewählt werden. Die Mengenangaben für den Photoinitiator schließen alle Initiatorbestandteile ein, wie z.B. Reduktionsmittel.

Besonders bevorzugt sind solche Adhäsive, die aus den genannten Stoffen bestehen. Weiterhin sind solche Adhäsive bevorzugt, bei denen die einzelnen Stoffe jeweils aus den oben genannten bevorzugten und besonders bevorzugten Stoffen ausgewählt sind.

Das Adhäsiv wird vorzugsweise in einem Multidosisbehälter oder besonders bevorzugt Einzeldosisbehälter untergebracht.

Ein Multidosisbehälter, wie z.B. einer Tropfflasche, enthält eine für mehrere Anwendungen ausreichende Adhäsivmenge und wird mit einem oder mehreren separaten Applikatoren kombiniert, wobei der Applikator mit einer für mehrere Anwendungen, vorzugsweise für eine Anwendung, ausreichende Menge an Vanadium(IV)salz und ggf. Sulfin- und/oder Ascorbinsäure beschichtet ist. Bei der Verwendung eines Multidosisbehälters wird die benötigte Menge des Adhäsivs z.B. in eine Tüpfelplatte getropft. Die Anzahl der Tropfen wird so bemessen, dass das Verhältnis von Adhäsiv zu Applikatorbeschichtung im gewünschten Bereich liegt, d.h. eine gute Härtung und Haftwirkung erzielt werden. Das Adhäsiv wird von der Tüpfelplatte mit dem beschichten Applikator aufgenommen und auf der zu behandelnden Zahnoberfläche eingerieben.

Alternativ kann das Adhäsiv in einem Multidosisbehälter untergebracht werden, aus dem mittels einer Dosiervorrichtung eine voreingestellte Menge des Adhäsivs entnommen werden kann, wie z.B. einem Stiftapplikator. Die Entnahme erfolgt vorzugsweise über eine mit dem Behälter verbundene, austauschbare Kanüle, die auf die beschriebene Weise beschichtet ist. Vorzugsweise ist die Kanüle mit einer solchen Menge an Vanadiumsalz und ggf. Sulfinsäure oder Sulfinsäurederivat beschichtet, die zur Härtung der voreingestellten Adhäsivmenge erforderlich ist. Die Kanüle ist vorzugsweise zumindest teilweise beflockt oder mit einem Schwamm, Borsten oder Gummilamellen versehen. Eine Kanüle kann für mehrere Anwendungen eingesetzt werden. Vorzugsweise wird zur Entnahme einer weiteren Portion aber eine neue Kanüle verwendet. Erfindungsgemäß geeignete Stiftapplikatoren werden in der US 2009/0060624 A1 beschrieben und sind kommerziell unter der Bezeichnung VivaPen^{®} von der Ivoclar Vivadent AG erhältlich. Pro Anwendung werden vorzugsweise 3 bis 15 mg des Adhäsivs abgeben. Der Stiftapplikator enthält vorzugsweise ca. 2 ml des Adhäsivs.

Erfindungsgemäß bevorzugt ist ein Dentaladhäsivset, bei dem das Adhäsiv und der Applikator räumlich getrennt in einem Einzeldosisbehälter untergebracht sind, wobei die Menge des Adhäsivs in dem Behälter so auf die Menge an Vanadium(IV)salz und ggf. Sulfin- und/oder Ascorbinsäure auf dem beschichteten Applikator abgestimmt ist, dass das Adhäsiv nach dem Mischen mit dem Applikator eine für die radikalische Härtung ausreichende Menge an Vanadium(IV)salz und ggf. Sulfinsäurederivat enthält. Durch vordosierte Portionen von Adhäsiv und Vanadiumsalz, die vom Anwender nur noch gemischt werden müssen, werden ungeeignete Mischungsverhältnisse und Anwendungsfehler vermieden und ein hohe Anwendungssicherheit gewährleistet. Als Einzeldosisbehälter eignen sich z.B. Flaschen mit einem Adhäsivreservoir, die zusätzlich den beschichteten Applikator so aufnehmen können, dass die Komponenten nicht unbeabsichtigt miteinander in Kontakt gelangen.

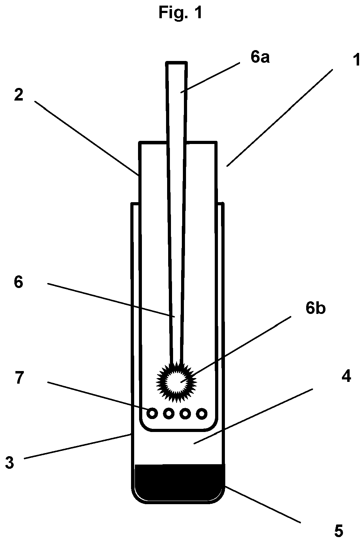

Ein beispielhafter Behälter ist in Figur 1 gezeigt. Der Behälter **1** umfasst das Oberteil **2** und das Gehäuse **3.** Das Oberteil **2** enthält den Applikator **6** mit dem Griffteil **6a** und dem Arbeitsende **6b.** Das Gehäuse **3** weist ein Reservoir **4** auf, das eine vorportionierte Menge des Adhäsivs **5** enthält. Das Gehäuse ist gegen das Eindringen von Fremdstoffen und die Verflüchtigung von Inhaltsstoffen durch geeignete Maßnahmen geschützt, z.B. durch eine Membran oder eine Dichtvorrichtung. Die Kombination von Einzeldosisbehälter und Applikator wird auch als Applikationsvorrichtung bezeichnet. Die Applikationsvorrichtung wird durch Einführen des Applikators in das Reservoir mit dem Adhäsiv aktiviert, beispielsweise durch das Durchstoßen einer Membran (nicht dargestellt) oder das Überwinden einer Dichtvorrichtung (nicht dargestellt) beim Hinabdrücken des Applikators. Bevorzugte Applikationsvorrichtungen werden in der EP 1 103 230 A2 und der EP 1 459 697 A2 beschrieben.

Die auf einen Applikator aufgebrachte Menge an Vanadyl(IV)salz und ggf. Sulfinsäure reicht zur Härtung von etwa 200 mg des Adhäsivs aus. Vorzugsweise wird die Adhäsivmenge so bemessen, dass im Adhäsiv eine Vanadyl(IV)salzkonzentration von 0,04 bis 2 Gew.-%, besonders bevorzugt 0,05 bis 1,3 Gew.-% und ggf. eine Sulfinsäurekonzentration von 0,08 bis 4 Gew.-%, besonders bevorzugt 0,1 bis 2,6 Gew.-% erzielt wird, jeweils bezogen auf die Adhäsivmenge.

Erfindungsgemäß bevorzugt sind Einzeldosisbehälter, die 90 bis 110 mg Adhäsiv enthalten. Diese werden vorzugsweise mit einem Applikator kombiniert, der mit 50 bis 80 µg Vanadyl(IV)salz beschichtet wird. Bei vollständigem Lösen des Vanadiumsalzes wird eine Vanadiumsalzkonzentration von 0,045 bis 0,09 Gew.-% im Adhäsiv erzielt. Bei Multidosisbehältern wird eine vorgegebene Adhäsivmenge mit einem Applikator gemischt und aufgenommen. Vorzugsweise werden etwa 30 mg Adhäsiv vorgelegt und mit dem Applikator aufgenommen, so dass ein mit 50 bis 80 µg Vanadiumsalz beschichteter Applikator zum Aufbringen mehrerer Portionen des Adhäsivs verwendet werden kann. Bei Stiftapplikatoren werden typischerweise 3 bis 15 mg Adhäsiv pro Anwendung entnommen, so dass auch hier ein mit 50 µg Vanadiumsalz beschichteter Applikator, beispielsweise eine Kanüle, für mehrere Anwendungen geeignet ist.

Der erfindungsgemäße beschichtete Applikator ermöglicht die Bereitstellung eines Adhäsivs in einer lagerstabilen Form. In Methacrylaten gelöste Vanadiumsalze und Sulfinate führen bereits in Anwesenheit von Sauerstoff, der aus der Luft in das Adhäsiv eindiffundiert, zur Radikalbildung und vorzeitigen Polymerisation des Adhäsivs. Erfindungsgemäß wird durch die Trennung von Vanadium(IV)salz und Adhäsiv eine vorzeitige Härtung des Adhäsivs verhindert. Erst unmittelbar vor der Applikation des Adhäsivs wird der Applikator mit dem Adhäsiv in Kontakt gebracht, wobei sich die Beschichtung vom Applikator abgelöst und im Adhäsiv verteilt. Auf diese Weise wird die destabilisierende Radikalbildung mit Luftsauerstoff umgangen, und es steht für jede Anwendung eine frisch hergestellte Adhäsivmischung zur Verfügung, die zuverlässig mit peroxidisch oder hydroperoxidisch initiierten Kompositen copolymerisiert.

Die erfindungsgemäßen Adhäsive eignen sich zur Anwendung mit peroxidisch und hydroperoxidisch initiierten Kompositen, d.h. Kompositmaterialien, die ein Hydroperoxid oder ein Peroxid als Initiator für die radikalische Polymerisation enthalten. Sie copolymerisieren auch ohne vorherige Lichthärtung gut mit den Kompositen und ergeben eine starke Verbundwirkung. Sie können aber auch zusammen mit lichthärtenden Kompositen eingesetzt werden, die z.B. bei der direkten Restaurationstherapie üblich sind. In diesem Fall enthalten die Adhäsive vorzugsweise einen Photoinitiator.

Das erfindungsgemäße Adhäsivset enthält vorzugsweise auch ein Kompositmaterial, das ein Peroxid oder vorzugsweise ein Hydroperoxid als Initiator für die radikalische Polymerisation enthält. Unter Kompositen werden Dentalwerkstoffe verstanden, die mindestens ein radikalisch polymerisierbares Monomer, vorzugsweise mindestens ein (Meth)arcylat, und mindestens einen Füllstoff enthalten. Besonders bevorzugt sind Komposite, die neben mindestens einem Hydroperoxid mindestens ein Thioharnstoffderivat enthalten.

Erfindungsgemäß bevorzugte Hydroperoxide sind Verbindungen der Formel R-(OOH)ₙ, in der R ein aliphatischer oder aromatischer Kohlenwasserstoffrest und n 1 oder 2 ist. Bevorzugte Reste R sind Alkyl- und Arylgruppen. Die Alkylgruppen können geradkettig, verzweigt oder cyclisch sein. Cyclische Alkylreste können durch aliphatische Alkylgruppen substituiert sein. Bevorzugt sind Alkylgruppen mit 4 bis 10 Kohlenstoffatomen. Arylgruppen können unsubstituiert oder durch Alkylgruppen substituiert sein. Bevorzugte aromatische Kohlenwasserstoffreste sind Benzolreste, die mit 1 oder 2 Alkylgruppen substituiert sind. Die aromatischen Kohlenwasserstoffreste enthalten vorzugsweise 6 bis 12 Kohlenstoffatome. Besonders bevorzugte Hydroperoxide sind t-Amylhydroperoxid, 1,1,3,3-Tetramethylbutylhydroperoxid, t-Butylhydroperoxid, t-Hexylperoxid, 2,5-Dimethyl-2,5-di(hydroperoxy)hexan, Diisopropylbenzolmonohydroperoxid, Paramenthanhydroperoxid, p-Isopropylcumolhydroperoxid und Mischungen davon. Ganz besonders bevorzugt ist Cumolhydroperoxid (CHP).

Bevorzugte Thioharnstoffderivate sind die in der EP 1 754 465 A1 in Absatz [0009] aufgeführten Verbindungen. Besonders bevorzugte Thioharnstoffderivate sind Acetyl-, Allyl-, Pyridyl- und Phenylthioharnstoff, Hexanoylthioharnstoff und Mischungen davon. Ganz besonders bevorzugt sind Acetylthioharnstoff (ATU) und Hexanoylthioharnstoff.

Weiter bevorzugt sind Thioharnstoffderivate mit der Formel 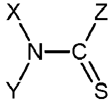 in er
- X: H oder Y ist,
- Y: ein Alkylrest mit 1 bis 8 Kohlenstoffatomen, ein Cycloalkylrest mit 5 oder 6 Kohlenstoffatomen, ein Chlor-, Hydroxy- oder Mercapto-substituierter Alkylrest mit 1 bis 8 Kohlenstoffatomen, ein Alkenylrest mit 3 bis 4 Kohlenstoffatomen, ein Arylrest mit 6 bis 8 Kohlenstoffatomen, ein Chlor-, Hydroxy-, Methoxy- oder Sulfonyl-substituierter Phenylrest, ein Acylrest mit 2 bis 8 Kohlenstoffatomen, ein Chlor- oder Methoxysubstituierter Acylrest, ein Aralkylrest mit 7 bis 8 Kohlenstoffatomen oder ein Chlor- oder Methoxy-substituierter Aralkylrest ist, und
- Z: NH₂, NHX oder NX₂ ist.

Die Komposite können darüber hinaus auch eine Übergangsmetallverbindung enthalten, die vorzugsweise aus den Verbindungen der Elemente Kupfer, Eisen, Kobalt, Nickel und Mangan ausgewählt ist. Bevorzugt sind Übergangsmetallverbindungen der Metalle Kupfer, insbesondere Cu⁺, Eisen, insbesondere Fe³⁺, Kobalt, insbesondere Co³⁺, und Nickel, insbesondere Ni²⁺. Die Übergangsmetalle werden bevorzugt in Form ihrer Salze eingesetzt. Bevorzugte Salze sind die Nitrate, Acetate, 2-Ethyl-hexanoate und Halogenide, wobei Chloride besonders bevorzugt sind.

Die Übergangsmetalle können auch in komplexierter Form eingesetzt werden, wobei Komplexe mit chelatbildenden Liganden bevorzugt sind. Bevorzugte einfache Liganden sind 2-Ethylhexanoat und THF. Bevorzugte chelatbildende Liganden sind 2-(2-Aminoethylamino)-ethanol, aliphatische Amine, besonders bevorzugt 1,1,4,7,10,10-Hexamethyltriethylentetramin (HMTETA), N,N,N',N",N"-Pentamethyldiethylentriamin (PMDETA), Tris[2-(dimethylamino)ethyl]amin (Me₆TREN), N,N,N',N'-Tetramethylethylendiamin (TMEDA), 1,4,8,11-Tetraaza-1,4,8,11-tetramethylcyclotetradecan (Me4CYCLAM), Diethylentriamin (DETA), Triethylentetramin (TETA) und 1,4,8,11-Tetraazacyclotetradecan (CYCLAM)); pyridinhaltige Liganden, besonders bevorzugt N,N,N',N'-Tetrakis(2-pyridylmethyl)ethylendiamin (TPEN), N,N-Bis(2-pyridylmethyl)amin (BPMA), N,N-Bis(2-pyridylmethyl)octylamin (BPMOA), 2,2'-Bipyridin und 8-Hydroxychinolin. Ganz besonders bevorzugte Liganden sind Acetylaceton, Dimethylglyoxim und 1,10-Phenanthrolin.

Bei elektrisch neutralen Liganden muss die Ladung der Übergangsmetallionen durch geeignete Gegenionen ausgeglichen werden. Hierzu kommen insbesondere die oben genannten Ionen in Betracht, die zur Bildung von Salzen verwendet werden, wobei Acetate und Chloride besonders bevorzugt sind. Chloride und Komplexe zeichnen sich durch eine relativ gute Löslichkeit in den Monomeren aus, die zur Herstellung der Dentalwerkstoffe eingesetzt werden.

Anstelle der Übergangsmetallkomplexe können nichtkomplexe Salze der Übergangsmetalle in Kombination mit komplexbildenden organischen Verbindungen zur Herstellung der Dentalwerkstoffe eingesetzt werden. Die organischen Liganden bilden beim Mischen mit den Übergangsmetallsalzen die katalytisch wirksamen Komplexe.

Bevorzugte Kupfersalze sind Cu(II)-carboxylate (z.B. der Essigsäure oder 2-Ethylhexansäure) CuCl₂, CuBr₂, Cul₂, besonders bevorzugt CuBr und ganz besonders bevorzugt CuCI. Bevorzugte Kupferkomplexe sind Komplexe mit den Liganden Acetylaceton, Phenanthrolin (z.B. 1,10-Phenanthrolin (Phen)), aliphatischen Aminen, wie z.B. 1,1,4,7,10,10-Hexamethyltriethylentetramin (HMTETA), N,N,N',N",N"-Pentamethyldiethylentriamin (PMDETA), Tris[2-(dimethylamino)ethyl]amin (Me₆TREN).

Bevorzugte Eisensalze sind FeCl₃, FeBr₂ und FeCl₂. Bevorzugte Eisenkomplexe sind Komplexe mit den Liganden Acetylaceton, Triphenylphosphin, 4,4'-Di(5-nonyl)-2,2'-bipyridin (dNbpy) oder 1,3-Diisopropyl-4,5-dimethylimidazol-2-yliden (Prilm). Ganz besonders bevorzugt sind die Komplexe Fe(acac)₂ und FeCl₂(PPh₃)₂.

Bevorzugte Nickelsalze sind NiBr₂ und NiCl₂, bevorzugte Nickelkomplexe sind Nickelacetylacetonat und NiBr₂(PPh₃)₂.

Erfindungsgemäß sind Kupferverbindungen, Kupferkomplexe und insbesondere Mischungen von Kupfersalzen und komplexierenden organischen Liganden bevorzugt, besonders Salze und Komplexe des einwertigen Kupfers (Cu⁺), wie z.B. Kupfer(I)chlorid (CuCI). Zusammensetzungen, die ein Salz des einwertigen Kupfers enthalten, zeichnen sich durch eine gute Lagerstabilität aus.

Erfindungsgemäß sind Komposite bevorzugt, die
(a) 0,001 bis 5,0 Gew.-%, bevorzugt 0,005 bis 3,0 Gew.-%, besonders bevorzugt 0,1 bis 3,0 Gew.-% mindestens eines Thioharnstoffderivats,
(b) 0,01 bis 5,0 Gew.-%, bevorzugt 0,05 bis 4,0 Gew.-% und besonders bevorzugt 0,1 bis 3,0 Gew.-% mindestens eines Hydroperoxids,
(c) 5 bis 95 Gew.-%, bevorzugt 10 bis 95 Gew.-% und besonders bevorzugt 10 bis 90 Gew.-% mindestens eines radikalisch polymerisierbaren Monomers,
(d) 0 bis 80 Gew.-% Füllstoff(e), und
(e) 0,01 bis 5 Gew.-%, bevorzugt 0,1 bis 3 Gew.-% und besonders bevorzugt 0,1 bis 2 Gew.-% Additiv(e) und ggf.
(f) 0,0001 bis 1 Gew.-%, bevorzugt 0,0005 bis 0,5 Gew.-%, besonders bevorzugt 0,0007 bis 0,02 Gew.-% mindestens einer Übergangsmetallverbindung enthalten.

Alle Mengenangaben hierin beziehen sich auf die Gesamtmasse der Zusammensetzung, wenn nicht anders angegeben. Als Monomere, Füllstoffe und Adhäsive sind die oben als Bestandteile des Adhäsivs genannten Komponenten bevorzugt.

Der Füllungsgrad richtet sich nach dem gewünschten Anwendungszweck des Werkstoffs. Füllungskomposite haben vorzugsweise einen Füllstoffgehalt von 50 bis 80 Gew.-%, besonders bevorzugt 70 bis 80 Gew.-%, und dentale Zemente von 10 bis 70 Gew.-%, besonders bevorzugt 60 bis 70 Gew.-%. Prothesenmaterialien haben vorzugsweise einen Füllstoffgehalt von 0 bis 10 Gew.-%, besonders bevorzugt 0 bis 5 Gew.-%,

Die erfindungsgemäßen Adhäsivsets eignen sich primär zur intraoralen Anwendung durch den Zahnarzt zur Restauration geschädigter Zähne, d.h. zur therapeutischen Verwendung. Sie sind besonders zur indirekten Restaurationstherapie geeignet, z.B. als adhäsive Zemente oder als Adhäsive zur Befestigung von Dentalrestaurationen aus Keramik, Glaskeramik, Metall oder gehärteten Kompositen am Zahn. Sie eignen sich aber auch zur gemeinsamen Anwendung mit Füllungskomposits für die direkte Restaurationstherapie. Sie können weiterhin extraoral (nicht therapeutisch) eingesetzt werden, beispielsweise bei der Herstellung oder Reparatur von Dentalrestaurationen wie Inlays, Onlays, Kronen und Brücken.

Die Erfindung wird im Folgenden anhand von Figuren und Ausführungsbeispielen näher erläutert.

Figur 1 zeigt einen zweiteiligen Einzeldosisbehälter **1** mit einem Oberteil **2** und einem unteren Gehäuse **3.** Das Gehäuse weist ein Reservoir **4** für das Adhäsiv **5** auf. Das Oberteil enthält einen beschichteten Applikator **6.** Das in Figur 1 gezeigte Oberteil **2** weist bei **7** Öffnungen auf, durch die das Adhäsiv **5** beim Eintauchen des Oberteils **2** in das Adhäsiv in das Oberteil eindringen kann.

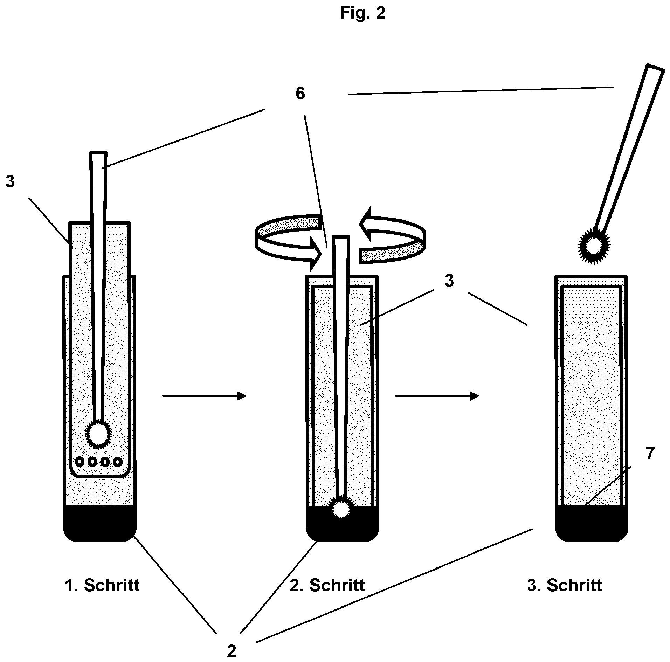

Figur 2 verdeutlicht die Anwendung des erfindungsgemäßen Adhäsivs. Vor Gebrauch werden die untere Hälfte **2** und die obere Hälfte **3** des Behälters zusammengeschoben und so der Applikator **6** mit dem Adhäsiv **5** in Kontakt gebracht (1. Schritt). Im zweiten Schritt wird der Applikator **6** in dem Adhäsiv **5** bewegt und so das gebrauchsfertige Adhäsiv **7** gebildet. Dieses kann im dritten Schritt mit dem Applikator **6** z.B. auf eine Zahnoberfläche aufgebracht werden.

### Ausführungsbeispiele

### Beispiele 1 bis 7:

### Herstellung beschichteter Applikatoren und Messung der Haftung

Zur Herstellung beschichteter Applikatoren wurden kleine Pinsel (Microbrush Tube Series, Hersteller Fa. Microbrush) mit der Spitze in die in Tabelle 1 aufgeführten Lösungen in der angegebenen Reihenfolge eingetaucht. Nach jedem Tauchschritt wurden die Pinsel für 5 bis 10 Minuten bei 40°C in einem Trockenschrank getrocknet. Anschließend wurden die Pinsel in ein Adhäsiv mit der in Tabelle 2 angegebenen Zusammensetzung (ca. 90 - 110 mg) eingetaucht, das Adhäsiv wurde für ca. 3 - 5 Sekunden mit dem Pinsel gerührt und dann auf Zahnoberflächen aufgebracht, für 20 s mit dem Pinsel auf der Zahnoberfläche einmassiert (Anpressdruck ausreichend für leichte Verformung des Pinselschafts) und mit einem Luftstrom (4 bar, ölfrei) aus einer Luftsprüheinheit für ca. 5 Sekunden getrocknet. Die Adhäsivschicht wurde vor dem Auftragen des Komposits nicht gehärtet.

Auf die mit Adhäsiv beschichteten Zahnoberflächen wurde dann ein zweikomponentiger, selbsthärtender peroxid- oder hydroperoxidhaltiger Komposit aufgebracht. Die Haftwerte wurden in Anlehnung an ISO 29022 auf bovinem Dentin ermittelt. Die Probevorbereitung und Prüfkörperdimensionen entsprachen der ISO 29022. Es wurden vorpolymerisierte Zylinder aus einem druckstabilen Dentalkomposit (Tetric EvoCeram, Höhe ca. 1,5 mm, Durchmesser ca. 2,37 mm) hergestellt, mit einem Sandstrahler (Renfert basic Quattro, 100 µm Aluminiumoxid, 1 bar) auf einer Stirnseite aufgeraut und mit dem zu testenden Befestigungskomposit auf die mit Adhäsiv vorbehandelte Zahnoberfläche gebracht. Durch ein senkrecht geführtes Gewicht von 500 g wurde sichergestellt, dass Zahnoberfläche und Zylinderlängsachse orthogonal zueinander angeordnet waren. Der Anpressdruck entlang der Zylinderlängsachse wurde für 15 Minuten unter Lichtausschluss bei Raumtemperatur gehalten. In dieser Zeit härtete der Komposit vollständig aus. Anschließend wurde der Probekörper für 24 h bei 37°C in Wasser aufbewahrt und wie in ISO 29022 beschrieben bis zum Bruch belastet. Die Bestimmung der Verbundstärke erfolgte gemäß ISO 29022 mit einer Universaltestmaschine vom Typ ZWICK-ROELL 010. Die gemessenen Haftwerte werden in Tabelle 1 aufgeführt.

Zur Bestimmung der Lagerstabilität wurden die beschichteten Applikatoren und das Adhäsiv für 2, respektive 8 Wochen bei 50°C eingelagert. In regelmäßigen Abständen wurden Proben genommen und wie oben beschrieben die Haftung auf Rinderzahndentin bestimmt. Die Werte für das hydroperoxidhaltige Komposit sind in Tabelle 1 angegeben.

Die Beispiele 1 bis 7 zeigen die Haftwerte, die mit erfindungsgemäß beschichteten Pinseln erzielt wurden. Es wurde sowohl mit peroxid- als auch mit hydroperoxidhaltigem Komposit gute Haftwerte erzielt. Die Beispiele 1 und 2 zeigen die Haftwerte von Pinseln, die erst mit dem Vanadiumsalz und dann mit einer Sulfinsäureverbindung beschichtet wurden. In diesen Beispielen wurden mit dem peroxidischen Komposit deutlich bessere Haftwerte als bei umgekehrten Beschichtungsreihenfolge (Beispiele 3 und 4) oder bei gemeinsamem Aufbringen von Vanadiumverbindung und das Sulfinat gemessen (Beispiele 5 und 6). Es wurde eine Haftung von über 9 MPa auf Schmelz und Dentin erreicht, was die für die klinische Anwendung mehr als ausreichend ist. Die Adhäsive zeichnen sich durch eine gute Lagerstabilität von 2, respektive 8 Wochen bei 50°C aus. Das Beispiel 7 zeigt, dass Vanadium(IV)oxalat auch ohne Sulfinat sowohl mit peroxid- als auch mit hydroperoxidhaltigen Kompositen hervorragende Haftwerte ergibt.

**Tabelle 1: Herstellung beschichteter Applikatoren und Haftwerte**

| **Bsp.** | **Tauchschritt 1** | **Tauchschritt 2** | **Dentin-Haftwerte** | | | |
|---|---|---|---|---|---|---|
| | | | **peroxidh. Comp.¹⁾** | **hydroperoxidhaltinger** | **Komposit²⁾** | |
| | | | **nach Herstellung** | **nach Herstellung** | **nach 2 Wo. @ 50°C** | **nach 8 Wo. @ 50°C** |
| **1** | Vanadylacetylacetonat/ Ascorbinsäure³⁾ | Natriumbenzolsufinat⁴⁾ | 13.5 ± 4.1 MPa | 32.6 ± 5.9 MPa | 18.2 ± 4.1 MPa | nicht bestimmt |
| **2** | Vanadylpicolinat/ Ascorbinsäure⁵⁾ | Natriumbenzolsufinat⁴⁾ | 10.2 ± 4.4 MPa | 29.2 ± 3.1 MPa | 19.3 ± 3.9 MPa | nicht bestimmt |
| **3** | Natriumbenzolsufinat⁴⁾ | Vanadylacetylacetonat/ Ascorbinsäure³⁾ | 3.9 ± 3.6 MPa | 28.8 ± 1.8 MPa | nicht bestimmt | nicht bestimmt |
| **4** | Natriumbenzolsufinat⁴⁾ | Vanadylpicolinat/ Ascorbinsäure⁵⁾ | 4.2 ± 2.2 MPa | 30.1 ± 2.7 MPa | nicht bestimmt | nicht bestimmt |
| **5** | Vanadylacetylacetonat/ Ascorbinsäure/ Natriumbenzolsufinat⁶⁾ | --- | 4.1 ± 2.8 MPa | 32.4 ± 1.6 MPa | nicht bestimmt | nicht bestimmt |
| **6** | Vanadylpicolinat/ Ascorbinsäure/ Natriumbenzolsufinat⁷⁾ | --- | 3.6 ± 4.2 MPa | 30.1 ± 2.0 MPa | nicht bestimmt | nicht bestimmt |
| **7** | Vanadyloxalat⁸⁾ | --- | 9.0 ± 1.1 MPa | 31.4 ± 3.3 MPa | 26.2 ± 3.0 MPa | 23.5 ± 6.7 MPa |

| | | | | | | |
|---|---|---|---|---|---|---|
| ¹⁾ Selbsthärtender peroxidhaltiger Komposit (Multicore Flow; Fa. Ivoclar Vivadent AG) ²⁾ Selbsthärtender hydroperoxidhaltiger Komposit (Variolink Esthetic DC; Fa. Ivoclar Vivadent AG) ³⁾ Lösung von 2 Gew.-% Vanadylacetylacetonat und 1 Gew.-% Ascorbinsäure in Methanol ⁴⁾ Lösung von 2 Gew.-% Natriumbenzolsufinat in Ethanol ⁵⁾ Lösung von 2 Gew.-% Vanadylpicolinat und 1 Gew.-% Ascorbinsäure in Methanol ⁶⁾ Lösung von 2 Gew.-% Vanadylacetylacetonat, 1 Gew.-% Ascorbinsäure und 2 Gew.-% Natriumbenzolsufinat in Methanol ⁷⁾ Lösung von 2 Gew.-% Vanadylpicolinat, 1 Gew.-% Ascorbinsäure und 2 Gew.-% Natriumbenzolsufinat in Methanol ⁸⁾ Lösung von 1 Gew.-% Vanadyloxalat in Methanol | | | | | | |

**Tabelle 2: Zusammensetzung des Adhäsivs**

| **Bestandteil** | **Gew.-%** | **Bezeichnung** |
|---|---|---|
| **a** | 12,50% | MDP⁴⁾ - Phosphatmethacrylate |
| | 4,33% | PO-25¹¹⁾ -Polyacrylsäuremethacrylat |
| **b** | 19,68% | HEMA¹⁾ - monofunktionelles Monomer |
| | 19,23% | bisGMA²⁾ - Vernetzer |
| | 8,65% | D3MA³⁾ - Vernetzer |
| **c** | 12,50% | Ethylalkohol |
| **d** | 11,57% | H₂O |
| **e** | 3,85% | Aerosil 200⁵⁾ |
| **f** | 0,17% | BHT⁶⁾ - Stabilisator |
| | 0,01% | MEHQ⁷⁾ - Stabilisator |
| | 0,01% | TEMPO¹²⁾- Stabilisator |
| | 3,85% | Kaliumhydroxid 2M in Wasser |
| **g** | 0,96% | Chivacure EPD⁸⁾ - Amin |
| | 1,73% | Campherchinon⁹⁾ (Photoinitiator) |
| | 0,96% | DMAEMA¹⁰⁾ - Aminomethacrylat |
| **Summe** | 100,00% | |

| | | |
|---|---|---|
| ¹⁾ 2-Hydroxyethylmethacrylat, CAS-Nr. 868-77-9 ²⁾ Bisphenol-A-glycerolatdimethacrylat, CAS-Nr. 1565-94-2 ³⁾ 1,10-Decanedioldimethacrylat, CAS-Nr. 6701-13-9 ⁴⁾ 10-Methacryloyloxydecyl-dihydrogenphosphat, CAS-Nr. 85590-00-7 ⁵⁾ pyrogene Kieselsäure mit einer spezifischen Oberfläche (BET) von 200 m²/g (CAS-Nr. 112 945-53-5; Fa. EVONIK) ⁶⁾ 2,6-Di-tert-butyl-4-methylphenol, CAS-Nr. 204-881-4 ⁷⁾ 4-Methoxyphenol, CAS-Nr. 150-76-5 ⁸⁾ Ethyl p-(dimethylamino)benzoate, CAS-Nr. 10287-53-3 ⁹⁾ Campherchinon, CAS-Nr. 10373-78-1 ¹⁰⁾ 2-(Dimethylamino)ethyl methacrylat, CAS-Nr. 2867-47-2 ¹¹⁾ Glycidylmethacrylat-modifizierte Polyacrylsäure, CAS-Nr. 54351-53-0 ¹²⁾ 2,2,6,6-Tetramethylpiperidinooxyl (TEMPO), CAS-Nr. 2564-83-2 | | |

## Patentansprüche

1. Dentaladhäsivset, das ein Adhäsiv und einen Applikator enthält, **dadurch gekennzeichnet, dass** der Applikator mit einem Vanadium(IV)salz beschichtet ist.

2. Dentaladhäsivset nach Anspruch 1, bei dem der Applikator mit Vanadyl(IV)oxalat und/oder Vanadyl(IV)acetylacetonat und/oder Vanadyl(IV)picolinat beschichtet ist.

3. Dentaladhäsivset nach Anspruch 1 oder 2, bei dem der Applikator zusätzlich mit mindestens einer Sulfinsäure und/oder einem Sulfinsäurederivat, vorzugsweise mit einem Sulfinsäureester und/oder Sulfinsäuresalz, besonders bevorzugt mit Natriumbenzolsulfinat und/oder Lithiumbenzolsulfinat beschichtet ist.

4. Dentaladhäsivset nach einem der Ansprüche 1 bis 3, bei dem der Applikator zusätzlich mit einem Reduktionsmittel beschichtet ist, vorzugsweise mit Ascorbinsäure oder einem Ascorbinsäurederivat.

5. Dentaladhäsivset nach einem der vorhergehenden Ansprüche, bei dem das Adhäsiv
(a) mindestens ein radikalisch polymerisierbares acides Monomer,
(b) mindestens ein radikalisch polymerisierbares Monomer ohne acide Gruppen,
(c) mindestens ein mit Wasser mischbares organisches Lösungsmittel,
(d) Wasser und ggf.
(e) ein oder mehrere Füllstoffe und ggf.
(f) einen oder mehrere Stabilisatoren und ggf.
(g) einen Photoinitiator für die radikalische Polymerisation und ggf.
(h) einen Verdicker enthält.

6. Dentaladhäsivset nach Anspruch 5, bei dem das Adhäsiv
(a) 1 bis 40 Gew.-%, bevorzugt 2 bis 30 Gew.-% und besonders bevorzugt 5 bis 20 Gew.-% acide radikalisch polymerisierbare(s) Monomer(e),
(b) 1 bis 80 Gew.-%, bevorzugt 1 bis 60 Gew.-% und besonders bevorzugt 5 bis 55 Gew.-% nicht acide radikalisch polymerisierbare(s) Monomer(e),
(c) 0 bis 70 Gew.-%, bevorzugt 5 bis 60 Gew.-% und besonders bevorzugt 10 bis 50 Gew.-% Lösungsmittel, vorzugsweise Methanol oder Ethanol,
(d) 0 bis 70 Gew.-%, bevorzugt 5 bis 60 Gew.-% und besonders bevorzugt 8 bis 50 Gew.-% Wasser und ggf.
(e) 0 bis 20 Gew.-% Füllstoff und ggf.
(f) 0,001 - 0,35 Gew.-%, bevorzugt 0,03 bis 0,30 Gew.-% und besonders bevorzugt 0,05 bis 0,25 Gew.-% Stabilisator(en) und ggf.
(g) 0,01 bis 10 Gew.-%, bevorzugt bis 0,1 bis 4,5 Gew.-% und besonders bevorzugt 0,5 bis 4,0 Gew.-% Photoinitiator und ggf.
(h) 0,1 bis 8 Gew.-%, besonders bevorzugt 0,2 bis 7 Gew.-% und ganz besonders bevorzugt 3 bis 6 Gew.-% Verdicker enthält,
jeweils bezogen auf die Gesamtmasse der Zusammensetzung.

7. Dentaladhäsivset nach einem der vorhergehenden Ansprüche, das zusätzlich ein Kompositmaterial enthält, das ein Hydroperoxid oder ein Peroxid als Initiator für die radikalische Polymerisation enthält.

8. Dentaladhäsivset nach Anspruch 7, bei dem der Applikator mit Vanadyl(IV)oxalat beschichtet ist und das Kompositmaterial ein Hydroperoxid als Initiator für die radikalische Polymerisation enthält.

9. Dentaladhäsivset nach einem der vorhergehenden Ansprüche, bei dem das Adhäsiv und der Applikator räumlich getrennt in einem Einzeldosisbehälter untergebracht sind, wobei die Menge des Adhäsivs auf die Menge an Vanadium(IV)salz und ggf. Sulfin- und/oder Ascorbinsäure auf dem beschichteten Applikator so abgestimmt ist, dass das Adhäsiv nach dem Mischen mit dem Applikator eine für die radikalische Härtung ausreichende Menge an Vanadium(IV)salz und ggf. Sulfinsäurederivat enthält.

10. Dentaladhäsivset nach einem der Ansprüche 1 bis 8, das einen Applikator und räumlich getrennt davon ein Adhäsiv enthält, das in einem Multidosisbehälter untergebracht ist, wobei die Menge an Vanadium(IV)salze und ggf. Sulfinsäurederivat auf dem beschichteten Applikator so bemessen ist, dass das Adhäsiv nach dem Mischen einer vorgegebenen Menge des Adhäsivs mit dem Applikator eine für die radikalische Härtung ausreichende Menge an Vanadium(IV)salz und ggf. Sulfinsäurederivat enthält.

11. Dentaladhäsivset nach einem der Ansprüche 1 bis 10 zur therapeutischen Verwendung bei der intraoralen Restauration geschädigter Zähne.

12. Verfahren zur Herstellung eines beschichteten Applikators mit den folgenden Schritten:
(1) Bereitstellen einer Lösung eines oder mehrerer Vanadium(IV)salze in einem geeigneten Lösungsmittel,
(2) ein- oder mehrfaches Eintauchen eines Applikators in die Lösung aus Schritt (1),
(3) Trocknen des Applikators aus Schritt (2) und vorzugsweise
(4) Bereitstellen einer Lösung einer Sulfinsäure und/oder eines Sulfinsäurederivats in einem geeigneten Lösungsmittel,
(5) ein- oder mehrfaches Eintauchen des Applikators in die Lösung aus Schritt (4) und
(6) Trocknen des Applikators aus Schritt (5).

13. Verfahren nach Anspruch 12, bei dem das Vanadium(IV)salz in Schritt (1) in Methanol gelöst wird, vorzugsweise in einer Konzentration 0,5 bis 5 Gew.-%, besonders bevorzugt 1 bis 2 Gew.-%, und bei dem die Sulfinsäure oder das Sulfinsäurederivat in Schritt (4) ist Ethanol gelöst wird, vorzugsweise in einer Konzentration 1 bis 10 Gew.-%, besonders bevorzugt ca. 2 Gew.-%.

14. Verfahren nach Anspruch 12 oder 13, bei dem in Schritt (1) neben dem Vanadium(IV)salz zusätzlich ein Reduktionsmittel, vorzugsweise Ascorbinsäure, in dem Lösungsmittel gelöst wird, vorzugsweise in einer Konzentration von 0,5 bis 5 Gew.-%, besonders bevorzugt ca. 1 Gew.-%.

15. Applikator, der mit einem Verfahren gemäß den Ansprüchen 12 bis 14 herstellbar ist.
